# EUROPEAN PATENT SPECIFICATION

(11) **EP 3 008 041 B1**
(45) Date of publication and mention of the grant of the patent: **18.09.2019**
(21) Application number: 14741689.5
(22) Date of filing: 10.06.2014
(51) Int. Cl.: C07D 213/82, A61K 31/455, A61P 9/10, A61P 7/02, A61P 3/00, A61P 9/12

(54) **3-CARBAMOYL-1-METHYLPYRIDINIUM NITRITE, PROCESS FOR ITS PREPARATION AND ITS USE**
3-CARBAMOYL-1-METHYLPYRIDINIUMNITRIT, VERFAHREN ZU SEINER HERSTELLUNG UND SEINE VERWENDUNG
NITRITE DE 3-CARBAMOYL-1-METHYLPYRIDINIUM, PROCEDE POUR SA PREPARATION ET SON UTLISATION

(30) Priority: 11.06.2013 PL 40427313
(43) Date of publication of application: 20.04.2016
(73) Proprietor: Uniwersytet Jagiellonski, 31007 Krakow (PL)
(72) Inventor: CHLOPICKI, Stefan, 31-138 Kraków (PL); PERNAK, Juliusz, 60-573 Pozna (PL); NIEMCZAK, Micha, 88-100 Inowroclaw (PL); KRAMKOWSKI, Karol, 15-684 Bialystok (PL); LESZCZYnSKA, Agnieszka, 15-161 Bialystok (PL); WALCZAK, Maria, 30-853 Kraków (PL)
(86) International application number: PCT/PL2014/050036
(87) International publication number: WO 2014/200373

(56) References cited:
- WO-A2-2005/067927
- V. Kumar, S. Malhotra: "Ionic Liquids as Pharmaceutical Salts: A Historical Perspective", IONIC LIQUID APPLICATIONS: PHARMACEUTICALS, THERAPEUTICS, AND BIOTECHNOLOGY (ACS SYMPOSIUM SERIES), vol. 1038, Chapter 1 2010, pages 1-12, XP002728879, DOI: 10.1021/bk-2010-1038.ch001 ISBN: 9780841225473 Retrieved from the Internet: URL:http://pubs.acs.org/doi/abs/10.1021/bk -2010-1038.ch001 [retrieved on 2014-08-25]
- DECKER R H ET AL: "Preparation of N<1>-methylnicotinamide chloride by Dowex-2 chloride", ANALYTICAL BIOCHEMISTRY, ACADEMIC PRESS INC, NEW YORK, vol. 3, no. 4, April 1962 (1962-04), pages 357-358, XP024819450, ISSN: 0003-2697, DOI: 10.1016/0003-2697(62)90122-7 [retrieved on 1962-04-01]
- STEPHEN M BERGE ET AL: "Pharmaceutical salts", JOURNAL OF PHARMACEUTICAL SCIENCES, AMERICAN PHARMACEUTICAL ASSOCIATION, WASHINGTON, US, [Online] vol. 66, no. 1, January 1977 (1977-01), pages 1-19, XP002675560, ISSN: 0022-3549

## Description

### Technical field

The invention relates to a novel pyridinium salt in the form of a 3-carbamoyl-1-methylpyridinium nitrite (MNANO₂) and the process of its preparation. The solution of the invention also relates to the use of a 3-carbamoyl-1-methylpyridinium nitrite for the manufacture of a vascular protective agent for the treatment or prevention of conditions or diseases associated with the dysfunction of vascular endothelium, failure of endothelial synthesis of nitric oxide (NO) and/or failure of endothelial synthesis of prostacyclin (PGI₂), including the manufacture of drugs for treating a number of diseases associated with the dysfunction of vascular endothelium.

The substance of the invention exhibits the antithrombotic effect, dependent upon the inhibition of platelet activation and inhibition of the coagulation system, as shown by *in vivo* tests, carried out on two groups of rats: normotensive and with renovascular hypertension.

### State of art

Pyridinium halides have been known since the early twentieth century thanks to Menschutkin, who described the synthesis of pyridine with alkyl chloride. 1 -Alkylpyridinium chlorides and bromides exhibit activity against bacteria and fungi and are representatives of cationic surfactants. There are two available chlorides: 1-dodecylpyridine chloride and 1-hexadecylpyridine chloride. They are ingredients of many commercial formulations. The exchange of a chloride or bromide anion for an inorganic or organic anion resulted in obtaining pyridinium salts. Pyridinium salts, whose melting point is below 100°C, were named pyridinium ionic liquids. The topic of ionic liquids is becoming more and more popular in both scientific and industrial circles. The presence of substituents in the pyridine ring of pyridine ionic liquids affects both their physicochemical and biological properties and determines their toxicity.

1-Carbamoyl-1-methylpyridinium chloride (MNACl) is a known compound and the main metabolite of nicotinamide (NA, vitamin PP, vitamin B3), which is a precursor for the synthesis of NAD and NADP. 1-Methylnicotinamide (MNA) is produced in the liver in a reaction catalysed by nicotinamide N-methyltransferase (Stanulovic et al., 1971). In the course of further chemical changes, MNA is transformed, using aldehyde oxidase, into 1-methyl-4-pyridone-5-carboxamide (4-PYR) or 1-methyl-2-pyridone-5-carboxamide (2-PYR). MNA and its metabolites are excreted in urine. MNA had been considered biologically inactive for many years. Analytical techniques of MNA measurements were developed to monitor the levels of MNA excreted in urea or located in plasma, and they were treated as a marker of PP vitamin availability in the organism (Holman and De Lange, 1949; Vivian et al., 1958). It has been recently shown that MNA administered locally is effective in the treatment of certain skin diseases (Wozniacka et al., 2005; Gebicki et al., 2003). It was also discovered that MNA, by the action of this molecule on vascular endothelium, stimulates endothelium to secrete endogenous prostacyclin (PGI₂) dependent upon COX-2 and shows thrombolytic and anticoagulative activity (Chlopicki et al., 2007). Therefore, endogenous MNA may act as a stimulator for the generation of endogenous PGI₂.

1-Alkoxymethyl-3-carbamoylpyridinium chlorides exhibit strong bactericidal and fungicidal activity (Pernak et al., 2001).

Polish patent application no. PL 358682 discloses a new medical use of nicotinamide adenine dinucleotide (NAD) and/or nicotinamide adenine dinucleotide phosphate (NADP) for the preparation of a pharmaceutical composition for topical administration to the skin to treat psoriasis and prevent the relapses of psoriasis. This application also includes a pharmaceutical formulation for topical application to the skin to treat psoriasis or prevent the relapses of psoriasis, comprising an active compound selected from nicotinamide adenine dinucleotide (NAD), nicotinamide adenine dinucleotide phosphate (NADP) or mixtures thereof in combination with a suitable pharmaceutically acceptable medium and the cosmetic use of NAD and/or NADP for skin care after the regression of psoriatic lesions.

In turn, Polish patent application no. PL 364348 also discloses the use of a group of pyridinium salts for the manufacture of a vascular protective agent for the treatment and/or prevention of conditions or diseases associated with the dysfunction of vascular endothelium, oxidative stress and failure of the endothelial synthesis of prostacyclin (PGI₂). The subject of the application also relates to a group to pyridinium salts intended for use in diet supplementation and the use of said derivatives for the manufacture of an agent intended for nutritional use to protect blood vessels in mammals in conditions or diseases associated with the dysfunction of vascular endothelium, oxidative stress and failure of the endothelial synthesis of prostacyclin (PGI₂).

As a result of research, it has been surprisingly found that salt in the form of a 3-carbamoyl-1-methylpyridinium nitrite not only enhances the stimulation of endogenous prostacyclin (PGI₂) excretion, which is known in the art for MNA, but additionally is a donor of bioactive nitric oxide (NO), being a new compound which, apart from favourable pharmacological activity, is also characterised by an unexpected pharmacokinetic profile.

### Detailed description of the invention

The essence of the invention is a pyridinium salt comprising an anion in the form of a nitrite and a cation in the form of a 3-carbamoyl-1-methylpyridinium, as shown in Formula 1:

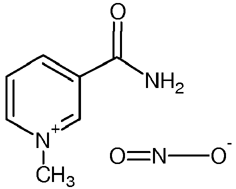

The present invention also provides the process for preparing 3-carbamoyl-1-methylpyridinium, which comprises the following steps:
a) 3-carbamoyl-1-methylpyridinium chloride, as shown in Formula 2: 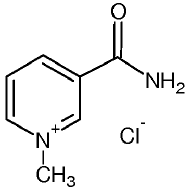 is dissolved in a short-chain alcohol, preferably in methanol or ethanol, mixed with sodium or potassium or lithium or ammonium nitrite, at a molar ratio of 3-carbamoyl-1-methylpyridinium to the nitrite from 1:0.8 to 1:1.2, preferably 1:1 at a temperature from 0°C to the boiling point of the solvent, most preferably at 45°C for at least 2 hours,
b) the inorganic by-product is then filtered out, and the solvent is removed from the filtrate.

3-Carbamoyl-1-methylpyridinium chloride is a substrate in the reaction of chloride anion exchange. The course of the reaction depends upon the developed conditions of synthesis. Laboratory practice shows that the conditions of synthesis, as well as the process of isolation of pure salt from the reaction mixture, should be individually developed for each new pyridinium salt derived from 3-carbamoyl-1-methylpyridine chloride.

The invention also relates to the compound of the invention for use in the treatment or prevention of conditions or diseases associated with the dysfunction of vascular endothelium, failure of endothelial synthesis of nitric oxide (NO) and/or failure of endothelial synthesis of prostacyclin (PGI₂), wherein the said condition or disease is selected from the following:
- atherosclerosis;
- atherosclerosis in patients with stable coronary artery disease, a cerebral ischemic episode or atherosclerosis of extremities, including thromboangiitis obliterans;
- hypercholesterolemia, hypertriglyceridemia or low HDL level;
- thrombosis due to reasons other than atherosclerosis, especially thrombosis associated with implantation of metallic vascular prostheses (stents), surgery of coronary artery bypass grafting (CABG), haemodialysis, thromboembolic disease of the veins;
- risk factors for atherosclerosis selected from a group consisting of the following factors: hypercholesterolemia, arterial hypertension, hyperhomocysteinemia, obesity, mental stress, infections, atherosclerosis of the transplant after organ transplantation and tolerance to nitrites;
- a group consisting of the following: chronic heart failure, pulmonary hypertension, nephrotic failure, chronic renal failure, adult acute respiratory distress syndrome (ARDS), cystic fibrosis, chronic obstructive pulmonary disease (COPD), preeclampsia - eclampsia, erectile dysfunction, Stein-Leventhal syndrome, sleep apnoea syndrome, systemic lupus erythematosus, sickle cell anaemia, stomach ulcers or duodenal ulcers, glaucoma, chronic liver disease, especially hepatitis, primary amyloidosis.

Using the solution of the invention permitted the obtaining of the following technical and economical results:
- the synthesised nitrite is characterised by a melting point of 82-84°C; it is a novel pyridinium ionic liquid,
- the obtained ionic liquid is soluble in water, methanol, ethanol, DMSO, is not soluble in hexane, diethyl ether, and it is thermally stable at temperatures up to 150°C,
- the synthesized ionic liquid is hygroscopic; it is necessary to keep it over a desiccant, preferably over P₄O₁₀,
- aqueous solutions of the obtained ionic liquid are not stable over time, which is observed as a change of their colour from pale yellow to brown,
- the preparation process is efficient and allows one to remove the by-product from the reaction mixture, and the reaction yield exceeds 95%,
- the synthesised 3-carbamoyl-1-methylpyridinium nitrite exhibits antithrombotic activity associated with the inhibition of platelets and inhibition of the plasma coagulation system.

MNANO₂, administered intravenously (*i.v.*) or intragastrically *per os* (*p.o.*), exhibits anticoagulative and antiplatelet activity without affecting the basic coagulation and morphological parameters, but it inhibits the generation of fibrin. Both MNA and NO₂, administered separately in a similar dose, do not show such a spectrum of pharmacological activity, which emphasises the importance of the invention.

### Brief description of figures in the drawing

The subject of the invention was described with the help of embodiments in the accompanying drawing, wherein:
- FIG. 1A: illustrates the dose-dependent antithrombotic activity of MNANO₂ *in vivo* in normotensive rats (single intravenous administration 10 minutes before the induction of arterial thrombosis).
- FIG. 1B: illustrates the antithrombotic activity of MNANO₂ *in vivo* in hypertensive rats compared to the results presented in FIG. 1A (the substance was administered to rats intravenously 10 minutes before the induction of arterial thrombosis or *per os* for 3 days twice a day).
- FIG. 2: illustrates the antiplatelet activity of MNANO₂ administered *ex vivo* (3 days of administration *per os* twice a day), resulting in the inhibition of thromboxane B₂ production in rats with hypertension and arterial thrombosis.
- FIG. 3: illustrates the platelet-independent antithrombotic activity of MNANO₂ administered *ex vivo* (single intravenous administration or administration *per os* for 3 days twice a day), resulting in the inhibition of fibrin production in rats with hypertension and arterial thrombosis.
- FIG. 4A and 4B: illustrate individual values of the concentration versus time dependence for MNA after single intravenous administration of MNANO₂ (4A) and MNACl (4B) at a dose of 100 mg/kg in rats (semi-logarithmic scale).
- FIG. 5A and 5B: illustrate individual values of the concentration versus time dependence for MNA after single intragastric administration of MNANO₂ (5A) and MNACl (5B) at a dose of 100 mg/kg in rats.

### Example 1: Synthesis of 3-carbamoyl-1-methylpyridinium (MNANO₂) nitrite

8.63 g (0.05 mole) of 3-carbamoyl-1-methylpirydinium chloride and 30 mL of methanol were introduced into a flask, and then 3.59 g (0.052 mole) of sodium nitrite was added. The reaction was conducted at 45°C for two hours under constant stirring. As a result of the anion exchange reaction, a white precipitate of sodium chloride appeared. The resulting inorganic salt was filtered out, and the methanol was evaporated under reduced pressure. The contents of the flask were then dissolved in anhydrous ethanol, and the residue of the insoluble excess of sodium nitrite was filtered out. The solvent, under reduced pressure, was evaporated out of the filtrate, and the 3-carbamoyl-1-methylpyridinium nitrate (III) was obtained in 98% yield. The synthesised ionic liquid is a yellow solid with a melting point of 82-84°C.

The structure of the compound was confirmed by a spectrum of proton and carbon nuclear magnetic resonance:
¹H NMR (400MHz, CDCl₃) δ [ppm] = 4.53 (s, 3H); 4.81 (s, 2H); 8.22 (t, *J* = 6.6 Hz, 1H); 8.93 (d, *J* = 9,3 Hz, 1H); 9.01 (d, *J* = 6,4 Hz 1H); 9.32 (s, 1H); ¹³C NMR (100MHz, CDCl₃) δ [ppm] = 168.55; 150.13; 147.93; 146.42; 136.29; 130.88; 51.42.

CHN elemental analysis for C₇H₉N₃O₃ (Mₘₒₗ = 183.16 g/mol): calculated values (%): C = 45.90; H = 4.95; N = 22.94; measured values: C = 45.67; H = 5.11; N = 22.76.

Thermal stability was determined using the TG technique under a nitrogen atmosphere (with the use of the Mettler Toledo Star TGA/DSC 1 device). The product after synthesis contained 6% of water, which was mainly adsorbed water. T_{onset5%} is 170°C and Tₒₙₛₑₜ = 195°C.

### Example 2: Pharmacological activity

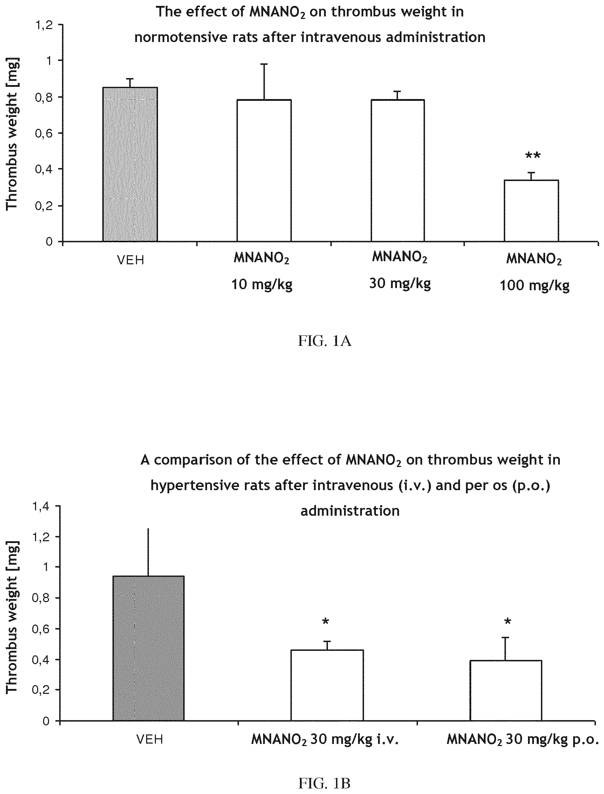

The anticoagulant effect of MNANO₂ was tested using a model of arterial thrombosis stimulated with an electrical current in the left carotid artery of normotensive and hypertensive rats (Schumacher et al., 1996). MNANO₂ was administered to rats intravenously (*i.v*.). Thrombus weight decreased in rats treated with MNANO₂ relative to the control group (Table 1, FIG. 1A), including especially the statistically important reduction of thrombus weight in the group in which the rats were treated with MNANO₂ in a dose of 100 mg/kg .

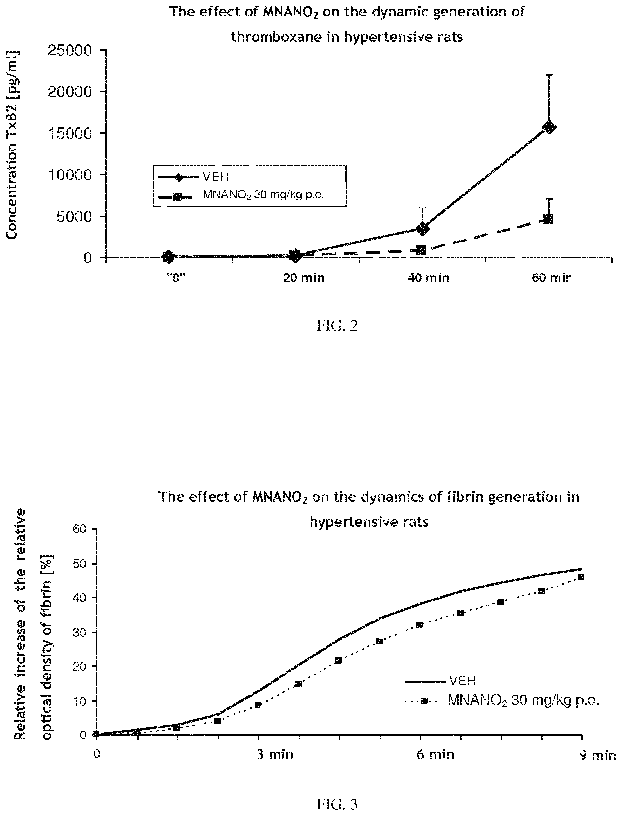

In addition, this effect was confirmed in hypertensive rats, and it was demonstrated that it does not depend upon the route of administration. In this case, MNANO₂ was administered to one group of rats intravenously (*i.v.*) and to the other group of rats intragastrically (*p.o.*). The results of the conducted tests indicate a reduction of thrombus weight in rats treated with MNANO₂ relative to the control group for both routes of administration (Table 2, FIG. IB). In the case of oral administration of MNANO₂ to rats, a reduction of fibrin generation was additionally observed (FIG. 3).

In turn, the antiplatelet activity of MNANO₂ was demonstrated by the inhibition of platelet activity assessed by the inhibition of thromboxane B₂ (TxB₂) production by platelets *ex vivo* (Luzak et al., 2012) in hypertensive rats after arterial thrombosis (FIG. 2).

Based on the above, it can be assumed that the anticoagulant activity of MNANO₂ depends upon the inhibition of platelets (FIG. 2). More importantly, MNANO₂ does not affect the coagulative parameters, such as PT (Table 1, Table 2); however, it inhibits the fibrin generation (Table 2, FIG. 3). In addition, based on automatic measurements in whole blood, it has been shown that MNANO₂ does not cause disorders of blood cell count parameters (Table 1, Table 2). Moreover, based on the chromatographic investigations of blood samples collected after thrombosis, it has been shown that MNANO₂ is a donor of MNA (Table 2).

**Table 1. Activity of MNANO₂ in normotensive rats; **p<0.01 vs VEH.**

| | VEH (n=15) | MNANO₂ 10 mg/kg *i.v.* (n=3) | MNANO₂ 30 mg/kg *i.v.* (n=3) | MNANO₂ 100 mg/kg *i.v.* (n=3) |
|---|---|---|---|---|
| Thrombus weight [mg] | 0.85±0.05 | 0.78±0,20 | 0.78±0.05 | 0.34±0.04** |

| Coagulation | | | | |
|---|---|---|---|---|
| APTT [s] | 24.58±1.21 | 2.00±2.68 | 25.67±6.07 | 26.27±2.89 |
| PT [s] | 20.14±0.48 | 18.23±0.68 | 18.10±0.81 | 19.17±0.27 |
| Fglevel [ng/mL] | 2.28±0.08 | 2.04±0.09 | 2.15±0.28 | 1.96±0.11 |
| QUICK | 52.90±3.40 | 61.75±5.42 | 63.00±7.57 | 53.33±1.67 |

| Complete blood cell count | | | | |
|---|---|---|---|---|
| WBC [106/µl] | 1.72±0.26 | 1.95±0.35 | 1.77±0.43 | 1.12±0.19 |
| RBC [106/µl] | 4.92±0.17 | 4.90±0.18 | 4.80±0.29 | 5.38±0.21 |
| HGB [g/dl] | 11.98±0.29 | 12.15±0.26 | 12.30±0.26 | 12.67±0.47 |
| HCT [%] | 29.84±1.18 | 30.03±1.26 | 29.07±1.61 | 33.33±1.31 |
| MCV [fl] | 60.60±0.81 | 61.25±0.25 | 60.68±0.33 | 62.00±1.16 |
| MCH [pg per cell] | 24.38±0.48 | 25.05±0.53 | 25.73±0.92 | 25.53±0.48 |
| MCHC [g/dl] | 40.36±1.11 | 40.93±1.00 | 42.40±1.32 | 37.97±0.18 |
| PLT [10³/µl] | 512±23 | 493±17 | 489±23 | 566±56 |

**Table 2. Activity of MNANO₂ in hypertensive rats; *p<0.05; ***p<0.001 vs VEH.**

| Substance | VEH (2K1C) n=13 | MNANO₂ 30 mg/kg *i.v.*; n=6 | MNANO₂ 30 mg/kg *p.o.*; n=4 |
|---|---|---|---|
| Thrombus weight [mg] | 0.94±0.33 | 0.46±0.06* | 0.39±0.18* |
| Concentration of MNA in plasma after collecting thrombus [nmol/mL] | 0.20±0.11 | 8.72±4.90*** | 5.76±3.78*** |

| Coagulation | | | |
|---|---|---|---|
| APTT [s] | 18.79±0.40 | 18.46±0.81 | 17.79±0.62 |
| PT [s] | 18.08±0.20 | 17.40±0.69 | 18.16±0.47 |
| Fglevel [ng/mL] | 2.56±0.07 | 2.36±0.13 | 2.61±0.10 |
| QUICK | 58.55±1.98 | 63.50±5.04 | 62.00±4.01 |
| Generation of fibrin (AUC) | 179.86±15.43 | 186.00±10.23 | 127.11±9.23* |

| Complete blood cell count | | | |
|---|---|---|---|
| WBC [106/µL] | 3.33±0.18 | 2.87±0.35 | 2.93±0.26 |
| RBC [106/µL] | 7.51±0.2 | 7.36±0.19 | 7.05±0.18 |
| HGB [g/dl] | 13.59±0.35 | 12.27±0.16 | 13.23±0.24 |
| HCT [%] | 41.58±1.07 | 38.35±0.49 | 40.07±0.03 |
| MCV [fl] | 55.42±0.31 | 54.86±0.60 | 57.00±1.53 |
| MCH [pg per cell] | 18.13±0.15 | 17.51±0.26 | 18.77±0.71 |
| MCHC [g/dl] | 32.68±0.15 | 31.65±0.21 | 32.97±0.58 |
| PLT [103/µL] | 623±24 | 621±18 | 630±12 |

### Induction and control of hypertension and subsequent induction of arterial thrombosis

The rats were anesthetised with pentobarbital (40 mg/kg, *i.p.*). The integument and muscles were cut to the height of the left kidney. The left renal artery was then partially ligated using a standardised clip. In such rats (n=60), renovascular hypertension (2K-1C, eng. 2kidney-1clip) developed within 6 weeks (Huang et al., 1981). The control group (n=6), serving to evaluate the development of hypertension, consisted of rats subjected to the same surgical procedure, but with no ligation of the renal artery (PO- virtually operated group). 6 weeks after the operation, in order to confirm the development of hypertension, blood pressure was measured by an indirect method in the awakened rats, as described by Zatz (1990). Further tests involved animals with a mean blood pressure (MBP) greater than 140 mmHg.

The normotensive and hypertensive rats were then anesthetised with pentorbital (40 mg/kg, *i.p.*). After removing the skin in the neck region, an approximately 0.5 cm long section of the left common carotid artery was isolated. To avoid haemorrhaging, the isolation was performed by tearing the surrounding tissues of the artery without the use of sharp instruments. The method of arterial thrombosis (according to Schumacher et al., 1996) with own modification (Kramkowski et al., 2012) involves damage to the endothelium isolated from a section of the artery by electric current (1 mA, 5-15 V, 10 min.). The anode is a stainless steel wire with a bent hook-shaped tip placed in a holder to remain stable during the experiment. Its tip was placed under the artery. A piece of parafilm was placed under the artery to keep it isolated. The cathode was a needle inserted subcutaneously in the hind limb region. Once the stimulation is completed, the formation of thrombus lasts 45 minutes. After this time, the artery was ligated at both sides of the thrombus, and the damaged fragment containing the thrombus was excised. The thrombus was quantitatively extracted from the artery and transferred to a previously tared microscope cover slip. The thrombi were allowed to dry (at room temperature and average humidity) and weighed not earlier than after 24 hours.

### Production of TxB₂ ex vivo

Citrated blood obtained from the right ventricle of hypertensive rats with arterial thrombosis which had been previously treated with MNANO₂ (or *vehiculum*) was used to evaluate the production of TxB₂ in an *ex vivo* experiment. The blood was diluted with 0.9% NaCl at a ratio of 1:1 and stirred in an aggregation cuvette at 1200 rpm. Samples of the stirred blood were collected after 20, 40 and 60 min and at "0" time and mixed with a cold solution of ASA (final concentration: 500 µM) at a ratio of 1:1. In these samples, TxB₂ was determined using the ELISA assay, according to the manual provided by the manufacturer of the reagent kit. The results for the concentration of TxB₂ *ex vivo* are shown in FIG. 2.

### Automatic evaluation of blood cell count and coagulation parameters of blood

The complete blood count was assessed approximately 2 minutes after collection in fresh citrated blood using an automatic Horiba ABC Vet device. The following parameters were assessed: white blood cell count (WBC), red blood cell count (RBC), haemoglobin (HGB), haematocrit (HCT), mean corpuscular volume (MCV), mean corpuscular haemoglobin (MCH), mean corpuscular haemoglobin concentration (MCHC) and platelet count (PLT).

Coagulation parameters: prothrombin time (PT), activated partial thromboplastin time (aPTT), fibrinogen concentration by Caluss and the prothrombin time percentage ratio (Quick index) were assessed in frozen plasma (-80°C) using a Caog-Chrom 3003 Bio-ksel Grudzi 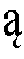 dz device. The plasma was extracted from citrated blood collected from the right ventricle of normotensive and hypertensive rats with arterial thrombosis which had been treated with MNANO₂ (or *vehiculum*).

The results of complete blood count and coagulation parameters are shown in Table 1 and Table 2.

### Example 3: Pharmacokinetic profile of MNANO₂

A method using high performance liquid chromatography combined with tandem mass spectrometry (LC/ESI-MS/MS) was developed and validated to determine concentrations of 1-methylnicotinamide (MNA) in rat plasma. The rats were given a single intravenous and intragastrical dose of 3-carbamoyl-1-methylpyrininium nitrite (MNANO₂) or, for comparison, MNA chloride (MNACl) at a dose of 100 mg/kg. The pharmacokinetic parameters were determined by a non-compartmental model using Phoenix WinNonlin software (Pharsight, Sannyvale, CA, USA).

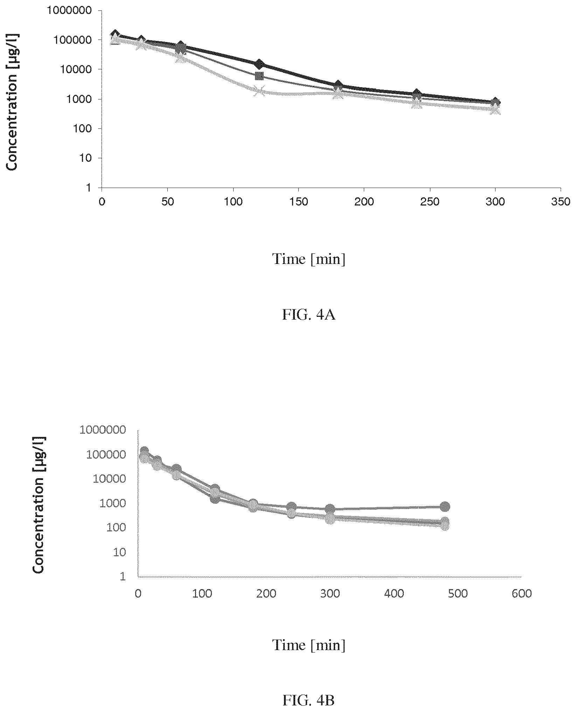

FIG.4A and FIG. 4B illustrate a profile of concentration change over time after single intravenous and intragastric administration of MNANO₂ and MNACl, respectively. In Table 3, the mean values of pharmacokinetic parameters in this system are summarised.

**Table 3. Mean values of the pharmacokinetic parameters of MNA determined in rat plasma after single intravenous administration of MNANO₂ and MNACl at a dose of 100 mg/kg, respectively.**

| | **Compound** | |
|---|---|---|
| **Parameter** | **MNANO₂** | **MNACl** |
| Co [µg/L] | 138,078 ± 33,208 | 141,311 ± 56,500 |
| AUC₀→∞ [µg·min/L] | 6,948,716 ± 1,810,664 | 4,362,231 ± 999,203 |
| MRT [min] | 42.4 ± 6.2 | 43.7 ± 9.81 |
| t_{0.5} [min] | 61.7 ± 19.6 | 162.5 ± 76.2 |
| Cl [L/min/kg] | 0.015 ± 0.003 | 0.024 ±0.005 |
| Vss [L/kg] | 0.63 ± 0.1 | 1.06 ± 0.35 |

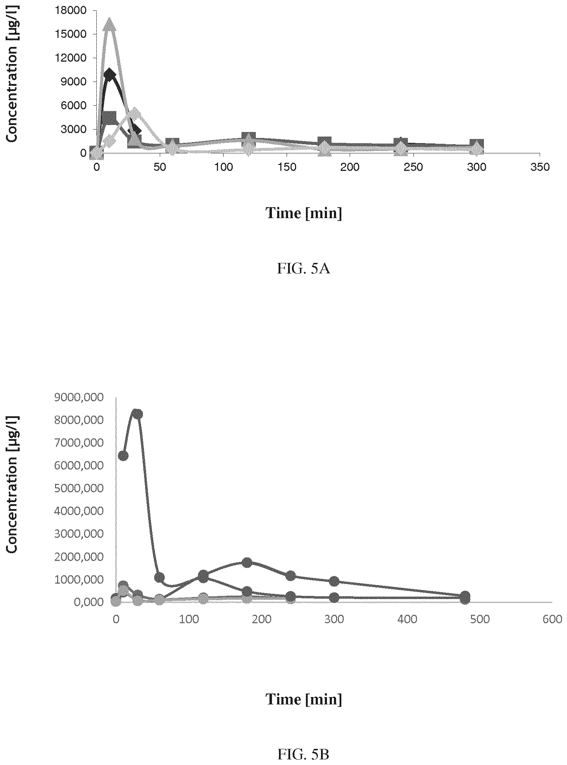

In turn, FIG. 5A and FIG. 5B illustrate individual values of the concentration versus time profile for MNA after single intragastric administration of MNANO₂ and MNACl at a dose of 100 mg/kg in rats. In Table 4, the mean values of the pharmacokinetic parameters in this system are summarised.

**Table 4. Mean values of the pharmacokinetic parameters of MNA determined in rat plasma after single intragastric administration of MNANO₂ and MNACl at a dose of 100 mg/kg.**

| **Parameter** | **Compound** | |
|---|---|---|
| | **MNANO₂** | **MNACl** |
| AUC₀→∞ [µg·min/L] | 638,056 ± 167,631 | 407,132 ± 164,906 |
| tmax [min] | 15 ± 10 | 57.5 ± 82.2 |
| Cmax [µg/L] | 8,881 ± 5,506 | 2,811 ± 3,669 |
| MRT [min] | 232 ± 38.3 | 358.7 ± 287.7 |
| t_{0.5} [min] | 178.4 ± 12.2 | 247.4 ± 201 |
| F [%] | 9.18 | 9.33 |

Based on the results obtained after a single intravenous administration of MNANO₂, it can be concluded that MNA is characterised by a relatively short half-life, which equals 61.7 ± 19.6 min, while for MNACl, it equals 162.5 ± 76.2 min. The mean residence time in the body is similar for both compounds and equals about 43 min. The volume of distribution for MNA after MNANO₂ administration equals 0.63 ± 0.1, and after MNACl administration, 1.06 ± 0.35 L /kg.

More importantly, after intragastric administration of MNANO₂, the concentration of the 3-carbamoyl-1-methylnicotinamide reaches a concentration three times higher in rat plasma compared to MNACl administration, and the time of occurrence of the maximum concentration is four times shorter. After intragastric administration of the same dose of MNANO₂ and MNACl, significant differences of the areas under the concentration-time curves (AUC), which are a measure of the amount of MNA in the body (about 2 times greater for MNANO₂ than for MNACl), are also visible. MNANO₂ is characterised by low bioavailability (9.18%), yet it is comparable to MNACl (9.33%).

To summarise, it can be concluded that the pharmacokinetic profile of MNA differs after MNANO₂ and MNACl administration; in particular, the process of adsorption after MNANO₂ administration is faster, and the observed concentrations of MNA in plasma are higher and are achieved within a significantly shorter time compared to MNACl.

### References cited in the description

1. Chlopicki S, Swies J, Mogielnicki A, Buczko W, Bartus M, Lomnicka M, Adamus J, Gebicki J: 1-Methylnicotinamide (MNA), a primarymetabolite of nicotinamide, exertsantithromboticactivitymediated by a cyclooxygenase-2/prostacyclinpathway. Br J Pharmacol 152:230-239 (2007).
2. Gebicki J, Sysa-Jedrzejowska A, Adamus J, Wozniacka A, Rybak M, Zielonka J. 1-Methylnicotinamide: a potentanti-inflammatory agent of vitaminorigin. Pol J Pharmacol 55:109-112 (2003).
3. Holman WI, De Lange DJ. Fate of N-methylnicotinamide in man. Nature 164:844 (1949).
4. Huang WC, Ploth DW, Bell PD et al. Bilateral renal function responses to converting enzyme inhibitor (SQ 20,881) in two-kidney, one clip Goldblatt hypertensive rats. Hypertension 3: 285-93 (1981).
5. Kramkowski K, Mogielnicki A, Leszczynska A, Buczko W. Angiotensin-(1-9), the product of angiotensin I conversion in platelets, enhances arterial thrombosis in rats. J Physiol Pharmacol. 61: 317-324 (2010).
6. Luzak B, Rywaniak J, Stanczyk L, Watala C. Pravastatin and simvastatin improves acetylsalicylic acid-mediated in vitro blood platelet inhibition. Eur J Clin Invest. 42:864-72 (2012).
7. Pernak J, Kalewska J, Ksycińska H, Cybulski J. Synthesis and anti-microbial activities of some pyridinium salts with alkoxymethyl hydrophobic group. Eur. J. Med. Chem. 36: 899-907 (2001).
8. Schumacher WA, Steinbacher TE, Megill JR, Durham SK. A ferret model of electrical-induction of arterial thrombosis that is sensitive to aspirin. J Pharmacol Toxicol Methods 35: 3-10 (1996).
9. Stanulovic M, Chaykin S. Metabolic origins of the pyridones of N1-methylnicotinamide in man and rat. Arch BiochemBiophys 145:35-42 (1971).
10. Vivian VM, Chaloupka MM, Reynolds MS. Some aspects of tryptophan metabolism in human subjects. I. Nitrogen balances, blood pyridine nucleotides and urinary excretion of N1-methylnicotinamide and N1-methyl-2-pyridone-5-carboxamide on a low-niacin diet. J Nutr 66:587-598 (1958).
11. Wozniacka A, Wieczorkowska M, Gebicki J, Sysa-Jedrzejowska A. Topical application of 1-methylnicotinamide in the treatment of rosacea: a pilot study. Clin Exp Dermatol 30:632-635 (2005).
12. Zatz R. A low cost tail-cuff method for the estimation of mean arterial pressure in conscious rats. Lab Anim Sci 40: 198-201 (1990).

## Claims

1. A pyridinium salt comprising an anion in the form of a nitrite and a cation in the form of a 3-carbamoyl-1-methylpyridinium, as shown in Formula 1:

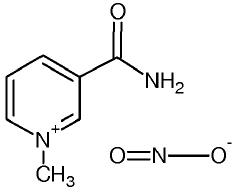

2. A process for preparing 3-carbamoyl-1-methylpyrininium nitrite, as shown in Formula 1, **wherein:**
a. 3-carbamoyl-1-methylpyridinium chloride, as shown in Formula 2: 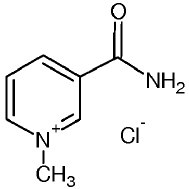 is dissolved in a short-chain alcohol, mixed with sodium or potassium or lithium or ammonium nitrite, at a molar ratio of 3-carbamoyl-1-methylpyridinium to the nitrite from 1:0.8 to 1:1.2, preferably 1:1 at a temperature from 0°C to the boiling point of the solvent.
b. the inorganic by-product is then filtered out, and the solvent is removed from the filtrate.

3. A process, according to claim 2, **wherein** the short-chain alcohol is methanol or ethanol.

4. A process, according to claim 2, **wherein** the molar ratio of 3-carbamoyl-1-methylpyridinium chloride to nitrite is 1:1.

5. A process, according to claim 2, **wherein** the reaction is carried out at 45°C for at least 2 hours.

6. The compound, according to claim 1, for use in the treatment or prevention of conditions or diseases associated with the dysfunction of vascular endothelium, failure of endothelial synthesis of nitric oxide (NO) and/or failure of endothelial synthesis of prostacyclin (PGI₂), wherein the said condition or disease is selected from the following:
a. atherosclerosis;
b. atherosclerosis in patients with stable coronary artery disease, a cerebral ischemic episode or atherosclerosis of extremities, including thromboangiitis obliterans;
c. hypercholesterolemia, hypertriglyceridemia or low HDL level;
d. thrombosis due to reasons other than atherosclerosis, especially thrombosis associated with implantation of metallic vascular prostheses (stents), surgery of coronary artery bypass grafting (CABG), haemodialysis, thromboembolic disease of the veins;
e. risk factors for atherosclerosis selected from a group consisting of the following factors: hypercholesterolemia, arterial hypertension, hyperhomocysteinemia, obesity, mental stress, infections, atherosclerosis of the transplant after organ transplantation and tolerance to nitrites;
f. a group consisting of the following: chronic heart failure, pulmonary hypertension, nephrotic failure, chronic renal failure, adult acute respiratory distress syndrome (ARDS), cystic fibrosis, chronic obstructive pulmonary disease (COPD), preeclampsia - eclampsia, erectile dysfunction, Stein-Leventhal syndrome, sleep apnoea syndrome, systemic lupus erythematosus, sickle cell anaemia, stomach ulcers or duodenal ulcers, glaucoma, chronic liver disease, especially hepatitis, primary amyloidosis.

## Patentansprüche

1. Das Pyridinsalz, das ein Anion in Form von Nitrit und ein Kation in Form von 3-Carbamoyl-1-methylpyridinium mit der Formel 1: 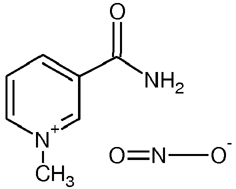 enthält.

2. Die Gewinnung von 3-Carbamoyl-1-methylpyridinium-Nitrit, wie in der Formel 1 angezeigt, wobei:
a. 3-Carbamoyl-1-methylpyridinium-Chlorid, wie in der Formel 2 angezeigt: 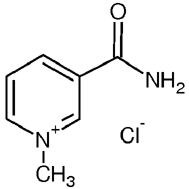 in kurzkettigem Alkohol löslich ist, sich mit dem Natrium- oder Kalium- oder Lithium- oder Ammoniumnitrit mischt, und zwar im molaren Verhältnis des 3-Carbamoyl-1-methylpyridinium-Chlorids zum Nitrit von 1:0,8 bis 1:1,2, bei Temperatur von 0°C bis zum Siedepunkt des Lösemittels;
b. ein anorganisches Nebenprodukt gefiltert wird, und das Lösemittel vom Filtrat entfernt wird.

3. Die Methode nach dem Patentanspruch 2, wobei ein kurzkettiger Alkohol Methanol oder Ethanol ist.

4. Die Methode nach dem Patentanspruch 2, wobei das molare Verhältnis des 3-Carbamoyl-1-methylpyridinium-Chlorids zum Nitrat 1:1 beträgt.

5. Die Methode nach dem Patentanspruch 2, wobei die Reaktion bei Temperatur von 45°C über wenigstens 2 Stunden verläuft.

6. Nach dem Patentanspruch 1 wird die Verbindung bei der Behandlung oder Vorbeugung von Zuständen oder Krankheiten, die mit der Dysfunktion des vaskulären Endothels, der gestörten Endothelsynthese von NO und/oder der gestörten Endothelsynthese von PGI₂ verbunden sind, verwendet, wobei der og. Zustand oder die og. Krankheit einer/eine der folgenden sein sollte:
a. Arteriosklerose;
b. Arteriosklerose bei Patienten mit einer stabilen koronaren Herzkrankheit, einem ischämischen Ereignis oder einer Extremitätensklerose, einschließlich der *Endangiitis obliterans;*
c. Hypercholesterinämie, Hypertriglyceridämie oder ein niedriger HDL-Spiegel;
d. Thrombose, die eine andere Ursache als Arteriosklerose hat, insbesondere Thrombose, die mit der Implantation von vaskulären Metallprothesen (Stents), koronaren Bypass-Operationen (CABG), der Hämodialyse, einer venösen Thromboembolie verbunden ist;
e. Risikofaktoren der Entwicklung der Arteriosklerose, die aus der folgenden Gruppe ausgewählt werden: Hypercholesterinämie, arterielle Hypertonie, Hyperhomocysteinämie, Fettleibigkeit, psychischer Stress, Infektionen, Sklerose der Gefäße des Transplantats bei der Transplantation der Organe und Nitrat-Toleranz;
f. Folgende Gruppe: chronische Herzinsuffizienz, Lungenhochdruck, nephrotisches Syndrom, chronische Niereninsuffizienz, akutes Lungenversagen (ARDS), Mukoviszidose, chronische obstruktive Lungenerkrankung (COPD), Präeklampsie - Eklampsie, Erektionsstörungen, Stein-Leventhal-Syndrom, Schlafapnoe-Syndrom, *Lupus erythematodes,* Sichelzellenanämie, Magen- oder Duodenumgeschwür, grüner Star, chronische Lebererkrankung, insbesondere Virushepatitis, primäre Amyloidose.

## Revendications

1. Sel de pyridinium contenant un anion sous forme de nitrite et un cation sous forme de 3-carbamyl-1-méthylpyridinium selon la Formule 1 :

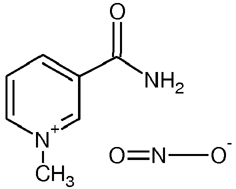

2. Procédé de préparation du nitrite de 3-carbamyl-1-méthylpyridinium, comme indiqué dans la Formule 1, mais :
a. chlorure de 3- carbamyl-1-méthylpyridinium, comme indiqué dans la Formule 2 : 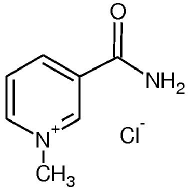 se dissout dans de l'alcool à chaîne courte, se mélange avec du sel de sodium ou de potassium ou de lithium ou d'ammonium du nitrite, dans le rapport molaire du chlorure de 3-carbamyl-1-méthylpyridinium au nitrite de 1:0,8 à 1:1,2, à température de 0 °C jusqu'à la température du point d'ébullition du solvant.
b. le sous-produit inorganique est évacué et le solvant est retiré du filtre.

3. Méthode selon la revendication 2, où l'alcool à chaîne courte est le méthanol ou l'éthanol.

4. Méthode selon la revendication 2, où le rapport molaire du chlorure de 3-carbamyl-1-méthylpyridinium au nitrate est de 1:1.

5. Méthode selon la revendication 2, où la réaction doit être effectuée à 45 °C pendant au moins 2 heures.

6. Substance selon la revendication 1, à utiliser dans le traitement ou la prévention d'affections ou de maladies liées au dysfonctionnement endothélial vasculaire, à la défaillance endothéliale d'oxyde nitrique (NO) et/ou à la synthèse endothéliale de prostacycline (PGI₂), où l'affection ou la maladie étant choisie parmi ce qui suit :
a. athérosclérose artérielle ;
b. athérosclérose des artères chez les patients présentant une coronaropathie stable, des épisodes ischémiques cérébraux ou une athérosclérose des membres, y compris une artérite thromboembolique ;
c. hypercholestérolémie, hypertriglycéridémie ou faible niveau de HDL ;
d. thrombose autre que l'athérosclérose des artères, en particulier thrombose associée à l'implantation de prothèses vasculaires métalliques (stents), pontage coronarien, hémodialyse, thromboembolie veineuse ;
e. facteurs de risque d'athérosclérose artérielle choisis parmi les les groupes suivants : hypercholestérolémie, hypertension, hyperhomocystéinémie, obésité, stress mental, infections, athérosclérose des vaisseaux transplantés et tolérance aux nitrates ;
f. groupe composé des éléments suivants : insuffisance cardiaque chronique, hypertension pulmonaire, syndrome néphrotique, insuffisance rénale chronique, syndrome respiratoire aigu de l'adulte (SDRA), fibrose kystique, maladie pulmonaire obstructive chronique (MPOC), éclampsie et pré-éclampsie, dysfonction érectile, syndrome de Stein-Leventhal, apnée du sommeil, lupus érythémateux disséminé, drépanocytose, ulcère gastrique ou duodénal, glaucome, maladie chronique du foie, en particulier hépatite virale, amyloidose primaire.
